# EUROPEAN PATENT APPLICATION

(11) **EP 3 905 260 A1**
(43) Date of publication of application: **03.11.2021**
(21) Application number: 20171866.5
(22) Date of filing: 28.04.2020
(51) Int. Cl.: G16H 50/30

(54) **METHOD, APPARATUS AND SYSTEM FOR TESTING BLOOD**

(71) Applicant: SYSMEX CORPORATION, Kobe-shi Hyogo 651-0073 (JP)
(72) Inventor: Bauer, Gabi, Hyogo, 651-0073 (JP); Klatte, Stefanie, Hyogo, 651-0073 (JP); Linssen, Joachim, Hyogo, 651-0073 (JP); Fuerst, Katharina, Hyogo, 651-0073 (JP)
(74) Representative: Hoffmann Eitle

(57) **Abstract**

Provided is a computer-implemented method for testing blood of a subject, comprising: obtaining a plurality of haematological parameters for the blood; performing a plausibility check of haematological parameters; and outputting an indication of a determined probability of an infectious response of the subject based on parameters according to a result of the plausibility check.

## Description

### Technical Field

The present invention relates to a computer-implemented method for testing blood, a diagnosis support apparatus, a blood cell counter system, and a computer program product.

### Background

Systemic Inflammatory Response Syndrome (SIRS) is a state where a subject is experiencing a serious inflammatory response in the whole body due to an infectious or non-infectious insult.

An example of SIRS accompanied by an infectious inflammatory response is sepsis. Sepsis is a disease that, if not appropriately treated at an early stage, the symptoms thereof can progress to serious sepsis, septic shock, and multiple organ dysfunction syndrome (MOD), ultimately leading to death. Therefore, if a subject has been given a diagnosis of SIRS, early determination of whether the response is an infectious inflammatory response or a non-infectious inflammatory response, in particular early diagnosis of whether the subject has a bacterial or viral infection has a great influence on the treatment to be given to the subject. Moreover, the determination of whether the inflammatory response is an infectious response or a non-infectious response may have a great influence on the treatment to be given to a subject who does not have SIRS.

### Summary of the Invention

### Technical Problem

EP 2 302 378 B1 describes a blood cell counter and diagnosis support method by which diagnosis support information is determined based on several measured haematological parameters, for example in the form of an index (ICIS: Intensive Care Infection Score), for supporting determination of whether an inflammatory response of a subject is an infectious inflammatory response, in particular an infectious inflammatory response caused by bacteria or virus. Bacterial or viral infection may rapidly progress to a life-threatening condition, such as sepsis, if not treated immediately and appropriately. A medical professional may attempt to determine if the inflammation is caused by a bacterial or viral infection, leading to a diagnosis of sepsis, or some other causes, leading to a diagnosis of SIRS.

The methodology of EP 2 302 378 B1 determines the ICIS index based on the measured haematological parameters and outputs the ICIS index as diagnosis support information. However, the ICIS index is outputted without a technical assessment of the reliability of the determined ICIS index, leading to the possibility of outputting inappropriate ICIS index values and to the possibility of an unreliable assessment of the likelihood of an infectious response such as sepsis.

Against this background, it is an object of the present invention to overcome these limitations.

### Solution

The object of the present invention is solved by the subject-matter as defined in the claims.

According to a first aspect, a computer-implemented method includes the features of claim 1. Preferred embodiments are defined in the corresponding dependent claims.

According to a second aspect, a diagnosis support apparatus includes the features of claim 16.

According to a third aspect, a blood cell counter system includes the features of claim 17.

According to a fourth aspect, a computer program product includes the features of claim 18.

The solution according to the first, second, third, and fourth aspects guarantees that only valid, reliable and numerical parameter values are used for determining the probability of an infectious response of the subject.

### Brief Description of the Drawings

FIG. 1 is a front view showing a schematic configuration of a blood cell counter system according to an embodiment.
FIG. 2 is a block diagram showing a configuration of a detection apparatus of the blood cell counter system according to the embodiment.
FIG. 3 is a schematic plan view showing a configuration of a detector of the detection apparatus according to the embodiment.
FIG. 4 is a block diagram showing a configuration of a first diagnosis support apparatus of the blood cell counter system according to the embodiment.
FIG. 5 is a block diagram showing a configuration of a second diagnosis support apparatus of the blood cell counter system according to the embodiment.
FIG. 6 is a flowchart showing a sequence of processing performed by the second diagnosis support apparatus of the blood cell counter system according to the embodiment.
FIG. 7 is a flowchart showing a sequence of processing performed by the second diagnosis support apparatus of the blood cell counter system according to another embodiment.
FIG. 8 shows a RET scattergram created by the detection apparatus of the blood cell counter system according to the embodiment.
FIG. 9 shows a DIFF scattergram created by the detection apparatus of the blood cell counter system according to the embodiment.
FIG. 10 shows a WBC/BASO scattergram created by the detection apparatus of the blood cell counter system according to the embodiment.
FIG. 11 shows an example of an output screen for showing a measurement result of the blood sample.
FIG. 12 shows another example of an output screen for showing a measurement result of the blood sample.
FIG. 13 is a flowchart showing a sequence of processing performed by the first diagnosis support apparatus of the blood cell counter system according to another embodiment.
FIG. 14 is a flowchart showing a sequence of processing performed by the first diagnosis support apparatus of the blood cell counter system according to another embodiment.
FIG. 15A and FIG. 15B show examples of DIFF scattergrams for explaining the plausibility check.
FIG. 16A and FIG. 16B show examples of RET scattergrams for explaining the plausibility check.

### Detailed Description of the Embodiment

Hereinafter, a specific description will be given of a diagnosis support apparatus, a diagnosis support method, and a computer program according to an embodiment of the present invention, with reference to the drawings. It will be understood that the embodiment below is not intended to limit the invention defined by the claims, and that all the combinations of the features described in the embodiment are not necessarily the essential matters for means for solution.

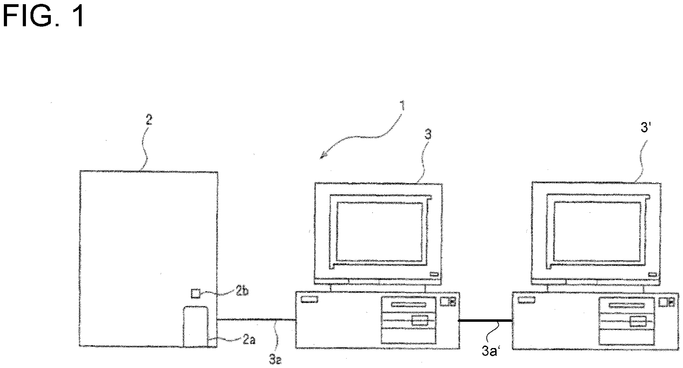

FIG. 1 is a front view showing a schematic configuration of the blood cell counter system according to the embodiment of the present invention. As shown in FIG. 1, the blood cell counter system 1 according to the embodiment of the present invention comprises a blood detection apparatus 2, a first diagnosis support apparatus 3 and a second diagnosis support apparatus 3'. The blood detection apparatus 2 is an apparatus that detects blood cells in the blood of a subject showing, for example, an inflammatory response. The first and second diagnosis support apparatuses 3 and 3' support a determination of whether the inflammatory response is caused by an infection or not. The first diagnosis support apparatus 3 may be a computer that may be referred to as Information Processing Unit (IPU) and that receives detection data from the blood detection apparatus 2 and analyses the detection data. The second diagnosis support apparatus 3' may be a computer that may be referred to as a work area manager (WAM) and that receives a measurement order for calculating ICIS index value of a subject from an external device (a laboratory information system (LIS)) and transmits the measurement order to the first diagnosis support apparatus 3.

The first diagnosis support apparatus 3 sends the measurement order of the subject to the detection apparatus 2 and receives data containing results of detection performed by the detection apparatus 2 and performs analysis processes. The blood cell counter system 1 is installed, for example, in a medical institution such as a hospital or a haematology laboratory. The detection apparatus 2 and the first diagnosis support apparatus 3 may be connected via a transmission cable 3a so as to be able to perform data communication there between. Likewise, the first diagnosis support apparatus 3 and the second diagnosis support apparatus 3' may be connected via a transmission cable 3a' so as to be able to perform data communication there between. Note, that the connection between the detection apparatus 2 and the first diagnosis support apparatus 3 and/or the connection between the first diagnosis support apparatus 3 and the second diagnosis support apparatus 3' is not limited to a direct wired connection formed by the transmission cable 3a and 3a', respectively. For example, the connection may be realized via a wireless connection such as a dedicated line using a telephone line, a LAN, or a communication network such as the Internet.

At the lower right of the front face of the detection apparatus (or blood analyser) 2, there is provided a blood collection tube setting part 2a on which a blood collection tube containing the blood of a subject can be set. When an operator presses a push button switch 2b provided near the blood collection tube setting part 2a, the blood collection tube setting part 2a moves toward the operator, thereby enabling the operator to set the blood collection tube thereon. When the operator presses the push button switch 2b again after setting the blood collection tube, the blood collection tube setting part 2a moves toward the detection apparatus 2 to be accommodated in the detection apparatus 2. However, the detection apparatus 2 is not limited to a manual setting of the blood collection tube. Alternatively, blood collection tubes may be collected in a sample rack and moved by a transporting band or transporting conveyor to the detection apparatus 2 where individual blood collection tubes are set to the detecting apparatus 2.

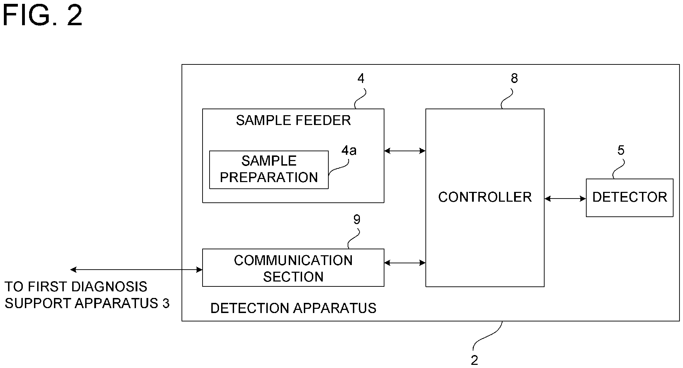

FIG. 2 is a block diagram showing a configuration of the detection apparatus 2 of the blood cell counter system 1 according to the embodiment of the present invention. Referring to FIG. 2, the detection apparatus 2 includes a sample feeder 4, a detector 5, a controller 8, and a communication section 9. The sample feeder 4 is a fluid unit including a sample preparation unit 4a composed of a chamber, a plurality of solenoid valves, a diaphragm pump, and the like. The sample preparation unit 4a prepares a detection sample by mixing the blood of a subject with a reagent. The sample feeder 4 feeds to the detector 5 a detection sample which is prepared by the sample preparation unit 4a. The controller 8 controls the operation of the components of the detection apparatus 2. The communication section 9 may be, for example, an RS-232C interface, a USB interface, or an Ethernet (registered trademark) interface, and transmits/receives data to/from the diagnosis support apparatus 3.

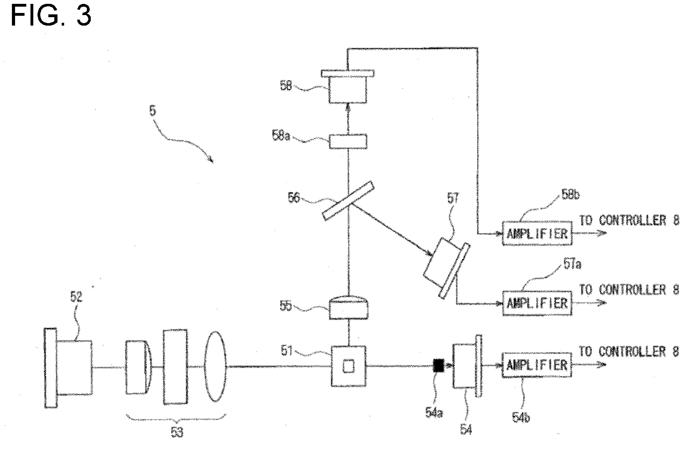

FIG. 3 is a schematic plan view schematically showing a configuration of the detector 5 of the blood cell counting system 1 according to the embodiment of the present invention. Referring to FIG. 3, the detector 5 is an optical flow cytometer, and detects white blood cells (WBC), reticulocytes (RET), mature red blood cells (RBC) and platelets (PLT) in blood by flow cytometry using a semiconductor laser. The term "red blood cell" is used herein as categorically including "reticulocyte (RET)" and "mature red blood cell (RBC)". The detector 5 includes a flow cell 51 for forming a liquid flow of the detection sample. The flow cell 51 is formed from a translucent material such as quartz, glass, synthetic resin, or the like, and has a tubular shape. The flow cell 51 has a flow path therein through which the detection sample and a sheath liquid flow. The detector 5 includes a semiconductor laser light source 52, which is disposed so as to output laser light toward the flow cell 51. Between the semiconductor laser light source 52 and the flow cell 51, an illumination lens system 53 including a plurality of lenses is provided. The illumination lens system 53 collects parallel beams outputted from the semiconductor laser light source 52 to form a beam spot. An optical axis extends linearly from the semiconductor laser light source 52 and through the flow cell 51. A photodiode 54 is provided on the optical axis, such that the photodiode 54 is located on the opposite side of the flow cell 51 from the illumination lens system 53. A beam stopper 54a is provided so as to block light coming directly from the semiconductor laser light source 52.

When a detection sample flows into the flow cell 51, scattered light and fluorescence occur based on the laser light. Of the scattered light and fluorescence, the light in the irradiation (i.e. forward) direction of the laser light is photoelectrically converted by the photodiode 54. Of the light traveling along the optical axis linearly extending from the semiconductor laser light source 52, the light coming directly from the semiconductor laser light source 52 is blocked by the beam stopper 54a. Incident on the photodiode 54 is only the scattered light that travels substantially along this optical axis direction (hereinafter referred to as forward scattered light). The forward scattered light emitted from the detection sample flowing in the flow cell 51 is photoelectrically converted by the photodiode 54 into electrical signals, and each photoelectrically converted electrical signal (hereinafter, referred to as a forward scattered light signal) is amplified by an amplifier 54b, to be outputted to the controller 8. The intensity of the forward scattered light signal indicates the size of a blood cell.

A side condenser lens 55 is provided laterally to the flow cell 51, so as to be located in a direction on an optical axis that crosses the optical axis that linearly extends from the semiconductor laser light source 52 to the photodiode 54. The side condenser lens 55 condenses side light (i.e. light that is outputted in the direction on an optical axis that crosses the optical axis that linearly extends from the semiconductor laser light source 52 to the photodiode 54) which occurs when laser light is emitted to the detection sample passing through the flow cell 51. A dichroic mirror 56 is provided downstream of the side condenser lens 55. The light condensed by the side condenser lens 55 is separated by the dichroic mirror 56 into scattered light components and fluorescence components. In an optical axis direction in which the light reflected by the dichroic mirror 56 advances (i.e. the direction on an optical axis that crosses the optical axis passing through the side condenser lens 55 and the dichroic mirror 56), a photodiode 57 for receiving the side scattered light is provided. On the optical axis that passes through the side condenser lens 55 and the dichroic mirror 56, an optical filter 58a and a photodiode 58 for receiving the fluorescence are provided.

The light reflected by the dichroic mirror 56 is the side scattered light, and is photoelectrically converted into electrical signals by the photodiode 57. Each photoelectrically converted electrical signal (hereinafter referred to as a side scattered light signal) is amplified by an amplifier 57a and then outputted to the controller 8. Each side scattered light signal indicates internal information of a blood cell (the size of the nucleus, etc.). The light transmitted through the dichroic mirror 56, which is the fluorescence, is photoelectrically converted into electrical signals by the photodiode 58 after being wavelength-selected by the optical filter 58a. Each photoelectrically converted electrical signal (hereinafter referred to as a fluorescence signal) is amplified by an amplifier 58b and then outputted to the controller 8. Each fluorescence signal indicates the degree of staining of a blood cell.

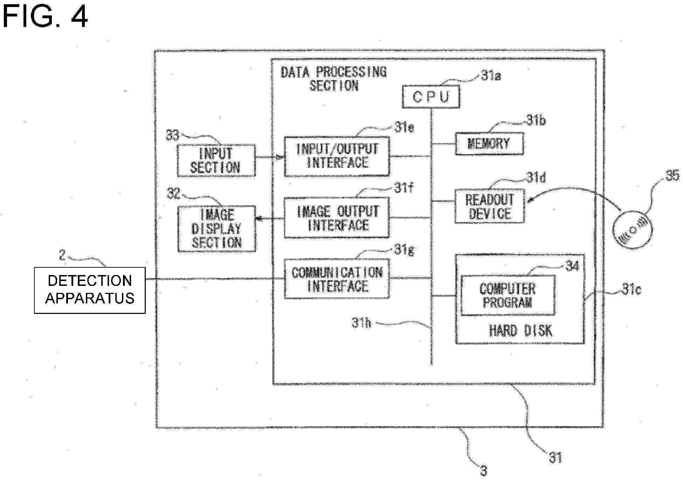

FIG. 4 is a block diagram showing a configuration of the first diagnosis support apparatus 3 of the blood cell counter system 1 according to the embodiment of the present invention. As shown in FIG. 4, the first diagnosis support apparatus 3 includes at least a data processing section (controller) 31 including a CPU (Central Processing Unit) and the like, an image display section 32, and an input section 33. The data processing section 31 includes a CPU 31a, a memory 31b, a hard disk 31c, a readout device 31d, an input/output interface 31e, an image output interface 31f, a communication interface 31g, and an internal bus 31h. In the data processing section 31, the CPU 31a is connected via the internal bus 31h to each of the memory 31b, the hard disk 31c, the readout device 31d, the input/output interface 31e, the image output interface 31f, and the communication interface 31g.

The CPU 31a controls the operation of each of the above hardware components and processes data received from the detection apparatus 2 in accordance with a computer program 34 stored in the hard disk 31c.

The memory 31b is structured as a volatile memory such as an SRAM, or a flash memory. To the memory 31b, a load module is loaded at the execution of the computer program 34. The memory 31b stores temporary data and the like which are generated at the execution of the computer program 34.

The hard disk 31c is structured as a fixed-type storage device or the like and is incorporated in the first diagnosis support apparatus 3. The computer program 34 is downloaded by the readout device 31d, which is a portable disc drive, from a portable storage medium 35 such as a DVD, a CD-ROM, a USB flash drive or the like that stores information, such as programs, data, and the like. The computer program 34 is then stored in the hard disk 31c. The computer program 34 is loaded from the hard disk 31c to the memory 31b so as to be executed. It will be understood that the computer program 34 may be a computer program downloaded via the communication interface 31g from an external computer.

The input/output interface 31e is connected to the input section 33 structured as a keyboard, a tablet, or the like. The image output interface 31f is connected to the image display section 32 which may be a CRT monitor, an LCD, or the like. Alternatively, the input section 33 and the image display section 32 may be included in a single device, such as a touch-based monitor.

The communication interface 31g is connected to the internal bus 31h and performs data transmission/reception with an external computer such as the second diagnosis support apparatus 3' (described below), the detection apparatus 2, or the like by being connected to an external network such as the Internet, a LAN, and a WAN. For example, the above hard disk 31c is not limited to the one incorporated in the first diagnosis support apparatus 3 but may be an external storage medium such as an external storage or the like that is connected to the first diagnosis support apparatus 3 via the communication interface 31g.

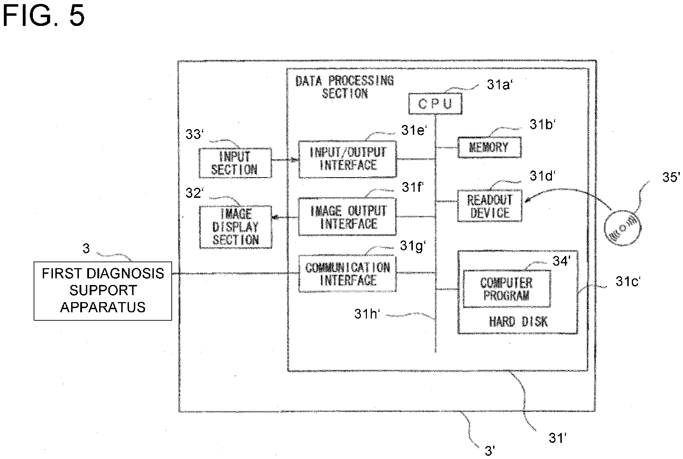

FIG. 5 is a block diagram showing a configuration of second diagnosis support apparatus 3' of the blood cell counter system 1 according to the embodiment of the present invention. The second diagnosis support apparatus 3' may have the same hardware configuration as the first diagnosis support apparatus 3 described above and a detailed explanation is therefore omitted. As shown in Fig. 5, the communication interface 31g' performs data transmission/reception with the first diagnosis support apparatus 3. Additionally, the hard disk 31c' may store a message indicating the ICIS index. The message may further include an indication that the subject has or probably has an infectious response, and a message indicating that the subject has or probably has a non-infectious response, as diagnosis support information for supporting an output of an indication of a determined probability of an infectious response of the subject (ICIS index). The hard disk 31c' may also store score thresholds (described below) for one or more of a plurality of haematological parameter, which are used for scoring the plurality of haematological parameter to determine whether the response of the subject is an infectious response or a non-infectious response, as well as thresholds for a plausibility check (described below).

Hereinafter, description is given of the operation of the blood cell counter system 1 according to the embodiment of the present invention. First, the sample feeder 4 of the detection apparatus 2 aspirates blood from a blood collection tube set in the blood collection tube setting part 2a, divides the aspirated blood into a plurality of aliquots according to a measurement order, and adds predetermined dedicated reagents to the aliquots, thereby preparing, for example, an RET detection sample, a DIFF detection sample, and WBC/BASO detection sample. Note that the RET detection sample is prepared by subjecting the blood to a dilution process and further to a staining process by use of a dedicated reagent for detecting reticulocytes. The DIFF detection sample is prepared by subjecting the blood to a dilution process, to a haemolyzing process by use of a dedicated reagent for haemolyzing red blood cells, and further to a staining process by use of a dedicated reagent for classifying white blood cells into multiple of sub-groups. The WBC/BASO detection sample is prepared by subjecting the blood to a dilution process, and further to a haemolyzing process by use of a dedicated reagent for haemolyzing red blood cells. The sample feeder 4 feeds the prepared detection samples to the flow cell 51 of the detector 5.

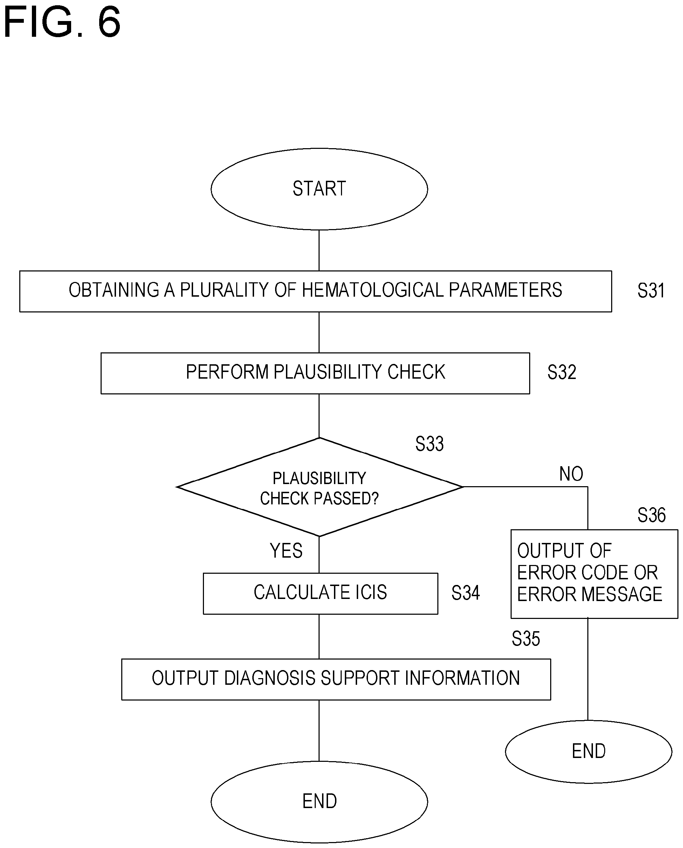

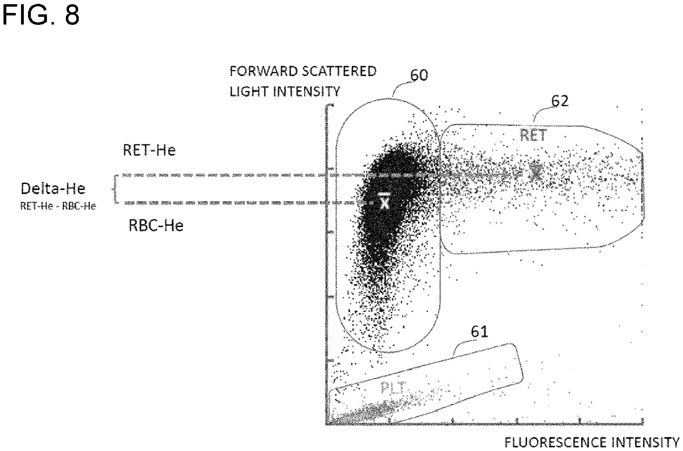

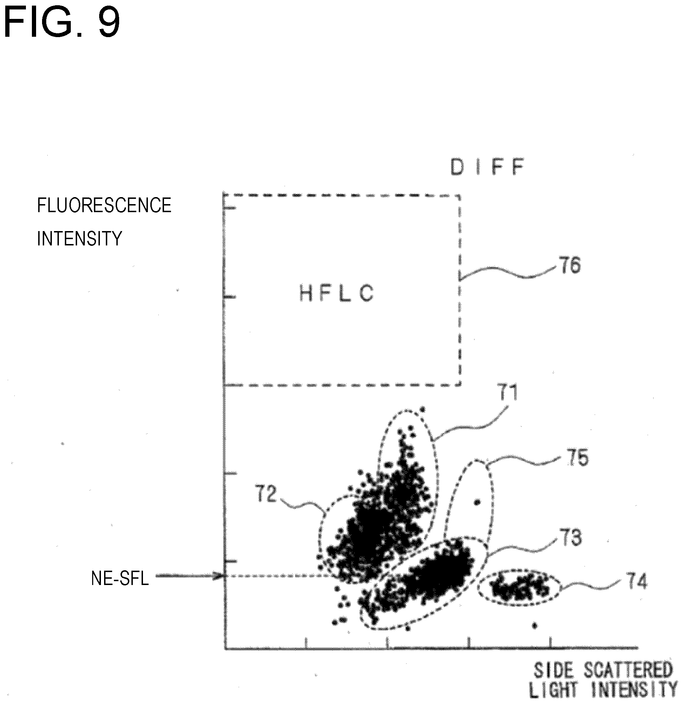

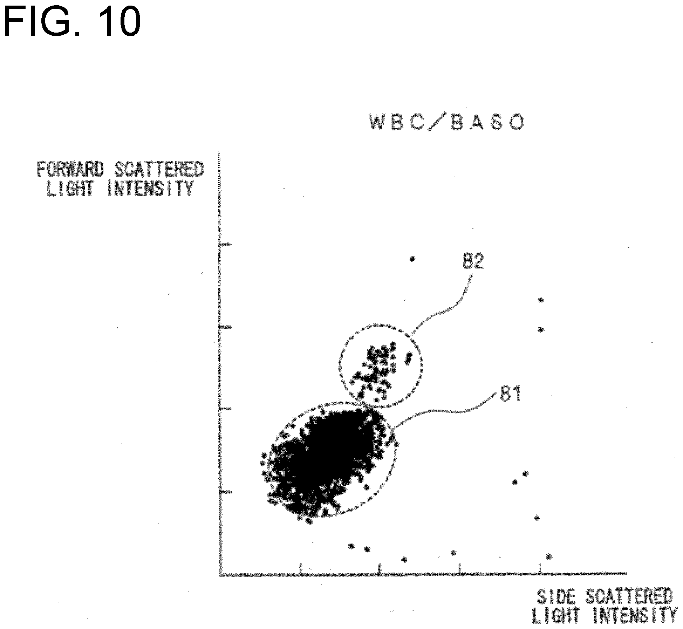

FIG. 6 is a flowchart showing a sequence of processing performed by the CPU 31a' of the data processing section 31' of the second diagnosis support apparatus 3' of the blood cell counter system 1 according to the embodiment of the present invention. As shown in FIG. 6, the CPU 31a' first obtains a plurality of haematological parameters, e.g. NEUT#, NE-SFL, RET#, HFLC#, IG#, EO#, Delta-He, RBC-He, PLT for the blood of the subject (S31). This may be achieved as follows:
When a detection sample is fed to the flow cell 51, the CPU 31a of the first diagnosis support apparatus 3 may receive via the communication interface 31g data of forward scattered light signals, side scattered light signals, and fluorescence signals outputted by the detector 5 of the detection apparatus 2 and stores the data in the memory 31b. The CPU 31a creates a plurality of scattergrams based on the data of the forward scattered light signals, the side scattered light signals, and the fluorescence signals detected by the detector 5 and stored in the memory 31b. The plurality of scattergrams created by the CPU 31a include, for example, at least a RET scattergram (see Fig. 8) having a Y-axis of the intensity of forward scattered light signals and an X-axis of the intensity of fluorescence signals, both of which signals are outputted by the detector 5; a DIFF scattergram (see Fig. 9) having a Y-axis of the intensity of fluorescence signals and an X-axis of the intensity of side scattered light signals, both of which signals are outputted by the detector 5; and a WBC/BASO scattergram (see Fig. 10) having a Y-axis of the intensity of forward scattered light signals and an X-axis of the intensity of side scattered light signals, both of which signals are outputted by the detector 5. Alternatively, the X-axis and the Y-axis of each scattergram may be reversed.

Next, the CPU 31a of the first diagnosis support apparatus 3 determines, by using the scattergrams as detection results, a plurality of haematological parameters for the blood of the subject. Examples of such haematological parameters are NEUT#, NE-SFL, RET#, HFLC#, IG#, EO#, Delta-He, RBC-He, PLT (as will be described in detail below).

FIG 8 shows a RET scattergram created by the blood cell counting system 1 according to the embodiment of the present invention. Based on the RET scattergram, the CPU 31a of the first diagnosis support apparatus 3 calculates the reticulocyte haemoglobin equivalent (RET-He), the difference (Delta-He) between the reticulocyte haemoglobin equivalent (RET-He) and the haemoglobin equivalent (RBC-He) of the mature red blood cells, both of which are obtained haematological parameters as follows. As shown in FIG 8, the CPU 31a of the first diagnosis support apparatus 3 identifies three areas: a mature red blood cell (RBC) area 60, a platelet (PLT) area 61, and a reticulocyte (RET) area 62 by use of the RET scattergram. Based on the RET scattergram, the CPU 31a determines an RBC-He, which is the median forward scattered light intensity of all the cells contained in the mature red blood cell area 60 (RBC), and a RET-He, which is the median forward scattered light intensity of all the cells contained in the reticulocyte area 62 (RET). The CPU 31a of the first diagnosis support apparatus 3 calculates a reticulocytes count (RET#) as one of the plurality of haematological parameters from the reticulocyte area 62. The CPU 31a of the first diagnosis support apparatus 3 further calculates a platelet count (PLT) as one of the plurality of haematological parameters from the platelet area 61. An example of such a calculation is shown in EP 2 302 378 B1.

Next, the CPU 31a of the first diagnosis support apparatus 3 calculates a neutrophil count (NEUT#) as one of the plurality of haematological parameters by using the DIFF scattergram and the WBC/BASO scattergram. Note that the term "granulocyte" is used herein as categorically including both of "mature granulocyte" and "immature granulocyte". The "mature granulocyte" categorically includes neutrophil (NEUT), eosinophil (EO), and basophil (BASO).

FIG. 9 shows a DIFF scattergram created in a white blood cell differential measurement by the blood cell counting system 1 according to the embodiment of the present invention. FIG. 10 shows a WBC/BASO scattergram created by the blood cell counting system 1 according to the embodiment of the present invention. As shown in FIG. 9, the DIFF scattergram classifies the blood cells into six areas of a monocyte (MONO) area 71, a lymphocyte (LYMPH) area 72, a neutrophil (NEUT) + basophil (BASO) area 73, an eosinophil (EO) area 74, an immature granulocyte (IG) area 75, and a highly fluorescent lymphocyte cell (HFLC) area 76. The sum of the number of the neutrophils (NEUT) and the number of the basophils (BASO) can be calculated by counting the number of the white blood cells in the neutrophil (NEUT) + basophil (BASO) area 73, based on the DIFF scattergram. Here, the CPU 31a of the first diagnosis support apparatus 3 further calculates an eosinophil count (EO#) as one of the plurality of haematological parameters from the eosinophil area 74.

In order to calculate the neutrophil count (NEUT#) as one of the plurality of haematological parameters from the sum of the number of the neutrophils (NEUT) and the number of the basophils (BASO) according to area 73 in FIG. 9, the CPU 31a of the first diagnosis support apparatus 3 determines the number of the basophils (BASO) by using the WBC/BASO scattergram. As shown in FIG. 10, the WBC/BASO scattergram classifies the white blood cells into two areas of a monocyte (MONO) + lymphocyte (LYMPH) + neutrophil (NEUT) + eosinophil (EO) area 81 and a basophil (BASO) area 82. Accordingly, the number of the basophils (BASO) in the basophil (BASO) area 82 can be calculated by counting the number of the white blood cells in the basophil (BASO) area 82, based on the WBC/BASO scattergram. The neutrophil count (NEUT#) as one of the plurality of haematological parameters can then be obtained by subtracting the number of the basophils (BASO) calculated based on the WBC/BASO scattergram from the sum of the number of the neutrophil (NEUT) and the number of the basophils (BASO) calculated based on the DIFF scattergram.

Next, the CPU 31a of the first diagnosis support apparatus 3 calculates a value indicating fluorescent light intensity of the neutrophils (NE-SFL) in the blood, which is one of the plurality of haematological parameters, by using the DIFF scattergram. Specifically, the value (NE-SFL) in FIG. 9 can be obtained by calculating, based on the DIFF scattergram, the median value of the fluorescence intensities of all the cells contained in the neutrophil (NEUT) + basophil (BASO) area 73 (i.e., neutrophils (NEUT) and basophils (BASO)). Although the obtained value (NE-SFL) includes an influence of fluorescence intensities of the basophils (BASO), the number of the basophils (BASO) is small and therefore the influence is small.

Next, the CPU 31a of the first diagnosis support apparatus 3 calculates an immature granulocyte count (IG#), which is one of the plurality of haematological parameters about the granulocytes in the blood, by using the DIFF scattergram. Specifically, the immature granulocyte count (IG#) can be obtained by counting the number of cells in the immature granulocyte (IG) area 75, based on the DIFF scattergram.

Next, the CPU 31a of the first diagnosis support apparatus 3 calculates a highly fluorescent lymphocyte cell count (HFLC#) about the antibody-synthesizing lymphocytes, which is one of the plurality of haematological parameters of the blood, by using the DIFF scattergram. Specifically, the highly fluorescent lymphocyte cell count (HFLC#) can be obtained by counting the number of cells in the highly fluorescent lymphocyte cell (HFLC) area 76, based on the DIFF scattergram.

The skilled person understands that the above example of obtaining a plurality of haematological parameters is non-limiting, and other scattergrams and other haematological parameters may be determined.

Referring back to Fig. 6, in step S31 the second diagnosis support apparatus 3' obtains the plurality of haematological parameters, e.g. NEUT#, NE-SFL, RET#, HFLC#, IG#, EO#, Delta-He, RBC-He, PLT, from the first diagnosis support apparatus 3 by receiving the determined haematological parameters (as described above) via the communication interface 31g'.

Then, according to step S32 of Fig. 6, the CPU 31a' of the second diagnosis support apparatus 3' performs a plausibility check with regard to at least one of the haematological parameters obtained in step 31.

In particular, the plausibility check may be performed with regard to a haematological parameter which is obtained from a white blood cell differential measurement such as NE-SFL and NEUT#. In addition, the plausibility check may be performed with regard to a haematological parameter for a white blood cell count, such as NEUT#, and/or a red blood cell count, such as RET#.

The plausibility check may be performed by comparing at least one of the haematological parameters with a threshold value or a lower threshold value and an upper threshold value. That is, the plausibility check may be performed by comparing a value of each of the haematological parameters used for the plausibility check with a corresponding threshold value or a corresponding lower and upper threshold. The threshold value(s) are predetermined and are set to guarantee that the haematological parameter has a reasonable or reliable value that fulfils a quality requirement with regard to the ICIS index value. In other words, the threshold value(s) are set to exclude implausible value that would lead to erroneous determination of the ICIS index value.

Next, in step S33 the CPU 31a' of the second diagnosis support apparatus 3' determines whether the plausibility check with regard to at least one of the haematological parameters has passed, i.e. whether or not the obtained at least one of the haematological parameter is above a predetermined threshold or is within a certain parameter range defined be a lower threshold value and an upper threshold value.

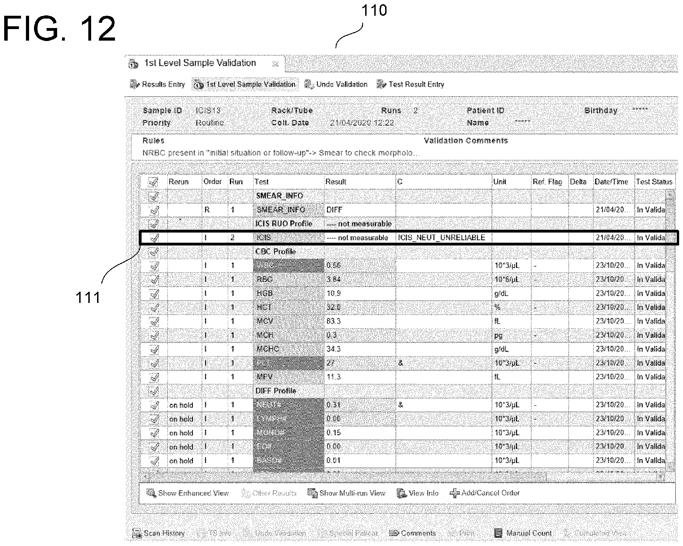

If the plausibility check is passed (Yes in step S33), the CPU 31a' of the second diagnosis support apparatus 3' continues with step S34 to determine an ICIS index value indicating a probability of having an infectious response, in particular indicating a likelihood of having a bacterial infection (as will be described in more detail below). On the other hand, if the plausibility check is not passed (No in step S33), the CPU 31a' continues with step S36 and outputs a corresponding error code or error message (as will be described in more detail below) while the indication of a determined probability (ICIS index) is not outputted. In addition, of the plausibility check is not passed (No in step S33), the CPU 31a' may cause an output of a measurement results of the at least one haematological parameter which causes a failure of the plausibility check (see below for FIG. 12).

When the plausibility check is passed the CPU 31a' determines an ICIS index value being related to a probability of having an infectious response, in particular being related to a likelihood of having a bacterial infection, based on the obtained haematological parameters (step S34) and then outputs the ICIS index value in step S35 as output diagnosis support information.

In particular, with regard to the calculation of the ICIS index value, the CPU 31a' may set ICIS points with regard to haematological parameters (Delta-He, RBC-He, NEUT#, EO#, PLT, NE-SFL, IG#, HFLC#) obtained in step S31, and calculates the ICIS index value by totalling the set ICIS points. This may be implemented by implementing parameter-specific ICIS rules as follows:

### a) ICIS rule with regard to HFLC#

Here, the CPU 31a' determines ICIS points based on the following rule with regard to haematological parameter HFLC#:

| Rule 1 | ICIS Points |
|---|---|
| HFLC# < M1/µL | 0 |
| M1/µL ≤ HFLC# < M2/µL | 1 |
| M2/µL ≤ HFLC# < M3/µL | 2 |
| HFLC# >= M3/µL | 4 |

The first ICIS rule applies specific score threshold values M1, M2, and M3. The skilled person understands that the actual numerical value of the threshold values M1, M2, and M3 depends on the blood sample, the configuration and accuracy of the detection apparatus 2 and the like. As such, depending on the determined highly fluorescent lymphocyte count HFLC# a corresponding first ICIS point 0, 1, 2 or 4 is set.

### b) ICIS rule with regard to IG# and EO#

Here, the CPU 31a' determines ICIS points based on the following rule with regard to haematological parameters IG# and EO#:

| Rule 2 | ICIS Points |
|---|---|
| IG# < M4/µL | 0 |
| M4/µL ≤ IG# < M5/µL | 1 |
| M5/µL ≤ IG# < M6/µL and EO# < M7/µL | 2 |
| IG# ≥ M6/µL and EO# < M7/µL | 4 |
| IG# ≥ M5/µL and EO# ≥ M7/µL | 0 |

The second ICIS rule applies specific score threshold values M4, M5, M6, and M7. The skilled person understands that the actual numerical value of the threshold values M4, M5, M6, and M7 depends on the blood sample, the configuration and accuracy of the detection apparatus 2 and the like. As such, depending on the determined immature granulocyte count IG# and determined eosinophil count EO# a corresponding second ICIS point 0, 1, 2 or 4 is set.

### c) ICIS rule with regard to NE-SFL

Here, the CPU 31a' determines ICIS points based on the following rule with regard to haematological parameter NE-SFL:

| Rule 3 | ICIS Points |
|---|---|
| NE-SFL < M8 ch | 0 |
| M8 ch ≤ NE-SFL < M9 ch | 1 |
| M9 ch ≤ NE-SFL < M10 ch | 2 |
| NE-SFL >= M10 ch | 4 |

The third ICIS rule applies specific score threshold values M8, M9, and M10. The skilled person understands that the actual numerical value of the threshold values M8, M9, and M10 depends on the blood sample, the configuration and accuracy of the detection apparatus 2 and the like. As such, depending on the determined median fluorescence value NE-SFL, a corresponding third ICIS point 0, 1, 2 or 4 is set.

### d) ICIS rule with regard to Delta-He and RBC-He

Here, the CPU 31a' determines ICIS points based on the following rule with regard to haematological parameters Delta-He and RBC-He:

| Rule 4 | ICIS Points |
|---|---|
| Delta-He ≥ M11 pg (picogram) | 0 |
| M12 pg ≤ Delta-He < M11 pg and RBC-He ≥ M13 pg | 1 |
| M14 pg ≤ Delta-He < M12 pg and RBC-He ≥ M13 pg | 2 |
| Delta-He < M14 pg and RBC-He ≥ M13 pg | 4 |
| Delta-He < M11 pg and RBC-He < M13 pg | 0 |

The fourth ICIS rule applies specific score threshold values M11, M12, M13, and M14. The skilled person understands that the actual numerical value of the threshold values M11, M12, M13, and M14 depends on the blood sample, the configuration and accuracy of the detection apparatus 2 and the like. As such, depending on the determined Delta-He and RBC-He values a corresponding fourth ICIS point 0, 1, 2 or 4 is set.

### e) ICIS rule with regard to NEUT#, EO#, and PLT

Here, the CPU 31a' determines ICIS points based on the following rule with regard to haematological parameters NEUT#, EO#, and PLT. With regard to the ICIS index, the total neutrophil granulocyte count may be understood as the IG# value and the NEUT# value together ("immature and mature granulocytes"), below referred to as NEUT#.

| Rule 5 | ICIS Points |
|---|---|
| M15/µL ≤ NEUT# < M16/µL | 0 |
| M16/µL ≤ NEUT# < M17/µL | 1 |
| NEUT# ≥ M17/µL and EO# < M18/µL and NEUT# < M19/µL | 2 |
| NEUT# < M15/µL | 2 |
| NEUT# ≥ M19/µL and PLT < M20/µL and EO# < M18/µL | 4 |
| NEUT# ≥ M17/µL and EO# ≥ M18/µL and NEUT# < M19/µL | 0 |
| NEUT# ≥ M19/µL and PLT ≥ M20/µL and EO# < M18/µL | 0 |
| NEUT# ≥ M19/µL and PLT < M20/µL and EO# ≥ M18/µL | 0 |
| NEUT# ≥ M19/µL and PLT ≥ M20/µL and EO# ≥ M18/µL | 0 |

The fifth ICIS rule applies specific score threshold values M15, M16, M17, M18, M19, and M20. The skilled person understands that the actual numerical value of the threshold values M15, M16, M17, M18, M19, and M20 depends on the blood sample, the configuration and accuracy of the detection apparatus 2 and the like. As such, depending on the determined NEUT#, EO#, and PLT values a corresponding fifth ICIS point 0, 1, 2 or 4 is set.

As explained, for calculating the ICIS index value in step S34, respective score threshold values are set with regard to the haematological parameters by applying a plurality of ICIS rules in which the haematological parameters obtained in step S31 are compared with respective score thresholds stored in advance in the hard disk 31c'. While EP 2 302 378 has further provided details as to how such score threshold values may be determined, for example based on a ROC (Receiver Operating Characteristic) analysis which uses ROC curves in order to determine score thresholds, the skilled person understands that other methods may be equally applied for the determination of the above score thresholds.

Accordingly, the ICIS index value may be calculated by the CPU 31a' by summing the respective ICIS points as determined according to the ICIS rules as described above. The minimum score is 0 points, which indicates that the inflammatory reaction is very unlikely caused by an infection. The maximum score is 20 points. The skilled person understands that the closer the score is to 20 points, the more likely the inflammatory reaction is caused by an infection such as a bacterial infection, as explained in EP 2 302 378 B1. The determination of the ICIS index is not limited to the above example. In particular, the skilled person understands that the determination of the ICIS index may be performed on the basis of more or less haematological parameters or other ICIS rules.

According to a further embodiment, the CPU 31a' of the second diagnosis support apparatus 3' may determine, based on the index (ICIS) calculated in step S34, a type of infectious response, e.g. whether the inflammatory response of the subject is an infectious response or a non-infectious response, for example an infectious inflammatory response or a non-infectious inflammatory response, or a bacterial infectious response or a non-bacterial infectious response. Then, the output diagnosis support information may include both the ICIS index and the type of infectious response.

In a further embodiment, the CPU 31a' may determine, based on the index (ICIS), whether the response of the subject is an infectious response or a non-infectious inflammatory response. In particular, the CPU 31a' may determine that the response of the subject is or probably is an infectious response if the index (ICIS) is not less than a determination threshold stored in the hard disk 31c; and determines that the response of the subject is or probably is a non-infectious response if the index (ICIS) is less than the determination threshold.

Description is now returned to the output process of the diagnosis support information performed by the CPU 31a' of the second diagnosis support apparatus 3' (see FIG. 6). If the plausibility check in step S33 is passed, the CPU 31a' outputs diagnosis support information via the image output interface 31f' to the image display section 32', and via the communication interface 31g' to another computer, printer, or the like (step S35). Specifically, the diagnosis support information includes the specific ICIS index value calculated in step S34 and may further include the determined type of infectious response.

Further, if the plausibility check is not passed, the CPU 31a' of the second diagnosis support apparatus 3' reads an error code or error message from the hard disk 31c' and outputs the error code or error message via the image output interface 31f' to the image display section 32', and via the communication interface 31g' to another computer, printer, or the like (step S36).

While the above embodiment has been described for the example of haematological parameters NEUT#, NE-SFL, RET#, HFLC#, IG#, EO#, Delta-He, RBC-He, PLT, the skilled person understands that the ICIS index value may be determined based on more or less haematological parameters, in particular that not all of the above haematological parameters have to be used for the ICIS index value. For example, in the above embodiment, while RET# is used for the plausibility check, it may not be required for determining the ICIS index value.

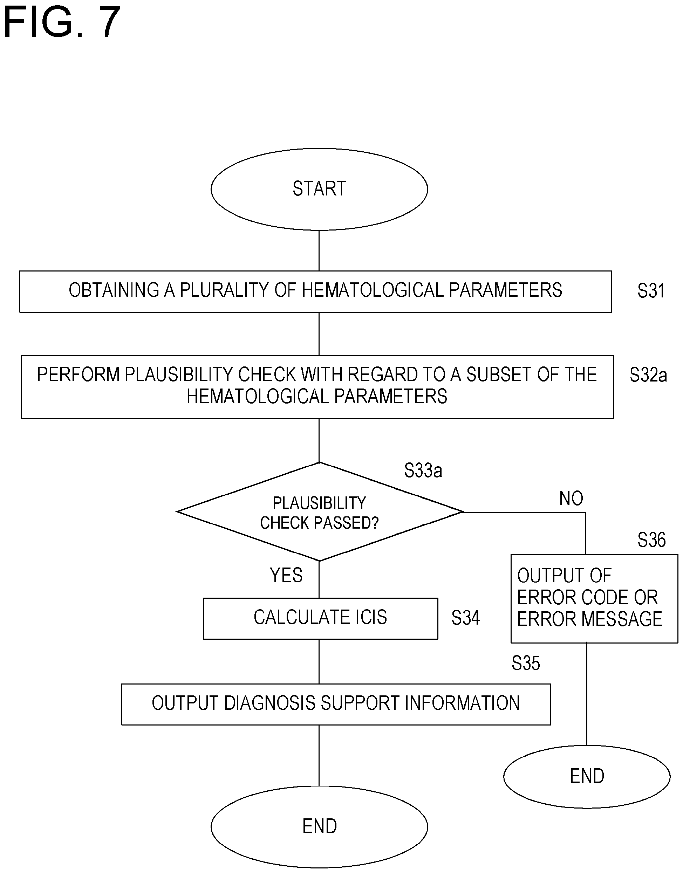

FIG. 7 is a flowchart showing a sequence of processing performed by the CPU 31a' of the data processing section 31' of the second diagnosis support apparatus 3' of the blood cell counter system 1 according to another embodiment of the present invention. Here, in difference to the processing sequence in Fig. 6, the plausibility check is performed in step S32a only with regard to a subset of the haematological parameters obtained in step S31. For example, if a set of 9 haematological parameters, such as NEUT#, NE-SFL, RET#, HFLC#, IG#, EO#, Delta-He, RBC-He, PLT is obtained in step S31, as explained above with regard to Fig. 6, then the plausibility check S32a does not have to be performed for the full set of haematological parameters, but can be performed only with a limited subset, for example with regard to the three parameters NEUT#, NE-SFL, RET#. The skilled person understands that this increases the efficiency of performing the plausibility check for the ICIS index of the blood sample, as a plausibility check is not required with regard to all obtained haematological parameters.

More specifically, according to step S32a of Fig. 7, the CPU 31a' of the data processing section 31' of the second diagnosis support apparatus 3' obtains a subset of haematological parameters and performs a plausibility check with regard to that subset. As explained above, when a plurality of haematological parameters such as NEUT#, NE-SFL, RET#, HFLC#, IG#, EO#, Delta-He, RBC-He, PLT is obtained, the subset of haematological parameters refers to a limited number of haematological parameters. In the present non-limiting example, this subset may correspond to the three parameters NEUT#, NE-SFL, RET#.

Here, the plausibility preferably has to be passed with regard to the entire subset of haematological parameters in order to output the diagnosis support information (ICIS index). In other words, if the plausibility check is successful (passed) only with, for example, one or two haematological parameters of the entire subset of haematological parameters but fails with regard to at least another haematological parameters of the entire subset, then the plausibility check of step 33a fails (is not passed).

The underlying reason for the possibility of having to perform the plausibility check only with regard to a limited subset of the obtained haematological parameters is based on the observations by the present inventors that there are relationships among the haematological parameters when determining plausibility. In particular, when considering the created scattergrams, it has been found that a plausible value for NEUT#, for example, also has a relationship to determining other haematological parameters to be plausible and vice versa.

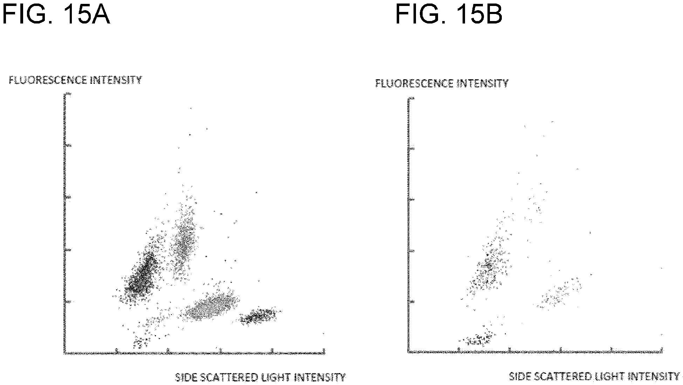

FIG.15A and FIG. 15B show an example of this relationship. In particular, according to the scattergram in Fig. 15A it is easy to determine a median value of NE-SFL (see above for FIG. 9), because there are clearly defined populations where this can be determined. FIG. 15B, however, shows a case of very low counts and the centre of neutrophil population may not be a real centre and the coefficient of variation between the scattergram dots is big, so that the NE-SFL is unreliable in case of very low NEUT#.

The skilled person understands that a failed plausibility check with regard to NE-SFL has an impact with regard to the other haematological parameters derived from the scattergram. For example, the reliability of the IG# classification or HFLC# (compare with FIG. 9) in general may be checked via NE-SFL which is a sensitivity parameter and reflects the fluorescence measure.

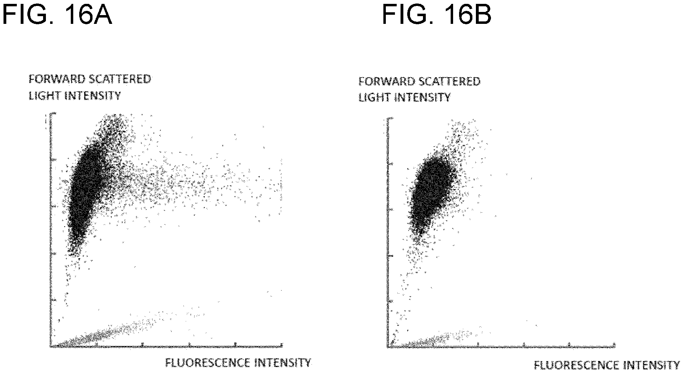

The reliability of Delta-He or RBC-He may be checked via RET# since these parameters are obtained from the same RET scattergram and RET# gives influence on the calculation of Delta-He and RBC-He. If the number of RET cells is below a threshold value, not enough cells are present and therewith no reliable centre of the RET population on the Y-axis can be determined which results in unreliable RET-He and therewith unreliable Delta-He. FIG. 16A and FIG. 16B show an example of this relationship, here for the RET scattergram. Here, FIG. 16A shows an example with a clear RET population. However, FIG. 16B shows an example in which are there (almost) no RET# present, so that the RET-He becomes unreliable. This unreliability then also relates to the haematology parameter Delta-He (RET-He - RBC-He), that is, if RET-He is unreliable, the Delta-He is also considered to be unreliable and therefore not considered for the ICIS index calculation.

In step S32a of FIG. 7, each parameter of the subset may be compared with a corresponding threshold value or a lower threshold value and an upper threshold value. In the above non-limiting example, the CPU 31a' is configured (specifically programmed) to check whether the RET# value is above a threshold value of, for example, a value reflecting a minimum RET population to obtain a reliable centre of the RET population and therewith RET-He value. The threshold value may be 0.004 10⁶/µL, 0.005 10⁶/µL or 0.006 10⁶/µL, etc. The skilled person understands that such a threshold value may be different for different sample sizes or different analyser setups.

If the RET# value is above this threshold value, the plausibility check with regard to RET# is passed (i.e. RET# has a plausible value). Otherwise, if the RET# value is equal to or less than this threshold value, then the CPU 31a' creates a parameter-specific message ("ICIS_RET_UNRELIABLE") as a code or flag indicating that the plausibility check for RET has failed which does not allow the calculation of the ICIS score.

Further, the CPU 31a' is configured (specifically programmed) to check whether the NE-SFL has a fluorescence intensity between a lower threshold and an upper threshold. If the fluorescence intensity is in a range defined between a lower threshold and an upper threshold, then the plausibility check with regard to NE-SFL is passed (i.e. NE-SFL has a plausible value). Otherwise, if the NE-SFL value is equal to or less than this lower threshold value or equal to or larger than this upper threshold, then the CPU 31a' creates a parameter-specific message ("ICIS_NE_SFL_UNRELIABLE") as a code or flag indicating that the plausibility check for NE-SFL has failed which does not allow the calculation of the ICIS score.

Further, the CPU 31a' is configured (specifically programmed) to check whether the NEUT# value is above a threshold value. Such a threshold value reflects a minimum NEUT population that is required to obtain a reliable NE-SFL (fluorescent light intensity of the NEUT area in the corresponding scattergram). If the NEUT# value is above this threshold value, the plausibility check with regard to NEUT# is passed (i.e. NEUT# has a plausible value). Otherwise, if the NEUT# value is equal to or less than this threshold value, then the CPU 31a' creates a parameter-specific message ("ICIS_NEUT_UNRELIABLE") as a code or flag indicating that the plausibility check for NEUT# has failed which does not allow the calculation of the ICIS score. The threshold value may be 0.4 10³/µL, 0.5 10³/µL or 0.6 10³/µL, etc. The skilled person understands that such a threshold value may be different for different sample sizes or different analyser setups.

Based on this plausibility check, the CPU 31a' determines in step S33a whether the plausibility check has passed with regard to all parameters of the subset, i.e. with regard to all three haematological parameters NEUT#, NE-SFL, RET# of the above example.

If the plausibility check has passed with regard to all parameters of the subset (Yes in step S33a), then the CPU 31a' continues with the ICIS index calculation in step S34 (as described in Fig. 6) and the remaining steps as explained above in Fig 7.

Otherwise, if the plausibility check is not passed with one or more parameters of the subset (No in step S33a), then the CPU 31a' continues with step S36 and outputs the error code (flag) or output message, e.g. ICIS_NEUT_UNRELIABLE, ICIS_NE_SFL_UNRELIABLE, ICIS_RET_UNRELIABLE, identifying which parameter of the subset has failed the plausibility check as described in Fig. 7.

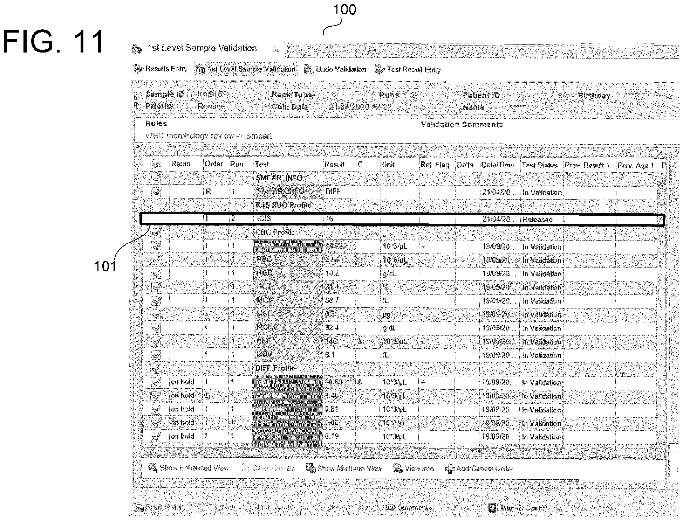

FIG. 11 shows an example of an output screen 100 for showing a measurement result of the blood sample according to a measurement order for calculating the ICIS index value in which the determined haematological parameters are indicated. Here, specific determined values of haematological parameters of the blood sample having a specific Sample ID, priority, collection date and the like, are shown in the output screen 100, for example with regard to NEUT#, LYMPH#, MONO# etc. In addition, the ICIS index value is shown by the highlighted box 101, which has a value of 16 for the present blood sample. From this value, the skilled person understands that the inflammatory reaction of the subject is highly likely to be an infectious response. As the ICIS index value is outputted as a consequence of the fact that the plausibility check has passed, a technically reliable ICIS index value can be provided.

FIG. 12 shows another example of an output screen 110 for showing a measurement result of the blood sample according to a measurement order for calculating the ICIS index value in which the determined haematological parameters are indicated, for example NEUT# = 0.31 10³/µL. Since the parameter for NEUT# has been obtained as 0.31 10³/µL and this value is below the corresponding threshold (as indicate above), the plausibility check has failed, no ICIS index value is determined (indicated as "not measurable" in the highlighted box 111 of Fig. 12) and it is indicated that the parameter NEUT# is unreliable for the ICIS index ("ICIS_NEUT_UNRELIABLE"). That is, although specific numerical values have been determined for the haematological parameter, the plausibility check still identifies the measurement result to be unreliable for the purpose of the ICIS calculation and therefore does not output an ICIS index value. As such, the error message or error code comprises information indicating at least one haematological parameter (here, with regard to the NEUT population) which causes a failure of the plausibility check. Note that, in the output screen 110, even if the ICIS index value is not displayed, the measurement result of the haematological parameter NEUT# which causes the failure of the plausibility check is displayed. This is because the NEUT# as such is considered to be correct and reliable. That is, the NEUT# is unreliable just for ICIS calculation because it is below the threshold value defined in the plausibility check, but the NEUT# as such is correct, so the NEUT# is displayed. The same applies for the RET measurement. In a case where there are no RET# present, the RET-He and the Delta-He become unreliable for ICIS calculation. But the RET# as such is correct, so the RET# is also displayed in the output screen.

The error message or error code may indicate the specific haematological parameter which causes a failure of the plausibility check. This enables an operator to grasp the cause of the non-execution of the ICIS calculation. For example, when the error message or error code indicates NE-SFL as the parameter which causes the failure of the plausibility check, the operator may conclude that the sensitivity of a fluorescence detector may be poor or a staining reagent may be deteriorated and thus take appropriate action.

In other embodiments the plausibility check in step S33 of Fig. 6 or step S33a of Fig. 7 may also be performed with regard to other conditions:
For example, the plausibility check may comprise checking a haematological parameter reflecting an operational status of the detection apparatus 2 that measures the blood. In particular, the haematological parameter NE-SFL reflects the sensitivity of detecting fluorescence from cells. As such, this haematological parameter may indicate the operational status of a fluorescence detector or a sample preparation device in the detection apparatus 2 for staining the cells with a fluorescence dye.

Alternatively or additionally, the plausibility check may comprise checking a parameter reflecting a quality of a reagent which is mixed with the. For example, the haematological parameter NE-SFL (being a sensitivity parameter which detects fluorescence activity in area 73 of FIG. 9) may also reflect the degree of staining of cells. This parameter may also indicate whether the staining reagent is deteriorated or not.

Alternatively or additionally, the plausibility check may comprise checking a specific haematological parameter whose value is related to obtaining reliable value of another haematological parameter among the plurality of haematological parameters. For example, the present inventors have recognized that a minimum NEUT population on the blood sample may be required to obtain a reliable NE-SFL. Similarly, the present inventors have also recognized that a minimum RET population may be required to obtain reliable RET-He and Delta-He parameters. This is because if the number of RET cells is below a threshold, not enough RET cells are present and therewith no reliable centre of the RET population on the Y-axis can be determined which results in unreliable RET-He and therewith unreliable Delta-He. Such intrinsic dependencies or relationships among the haematological parameters allow the plausibility test to be performed with regard to a reduced set of haematological parameters, such as the subset explained above, which makes the plausibility test more time efficient.

Moreover, the plausibility test may not only be performed with regard to the actual obtained (measured) haematological parameters of the blood of the subject but also with regard to the general conditions of the blood cell counter system 1 as well as the used reagents and thus may include an additional general plausibility test with regard to the used apparatus. Examples of such a general plausibility test may include one or more of a message format error (indicating that the format of a message from the haematology analyser (detection apparatus 2) is invalid, an aspiration error, an error flag, a function error, a suspect sample, a missing test, and/or an unsupported parameter unit (indicating that a result from the haematology analyser (detection apparatus 2) contains a unit for a blood test which is not supported for the calculation of the ICIS score.

For each of the above general plausibility checks, a corresponding individual error code may be provided. For example, "ICIS_Error_Result" indicates an erroneous measurement of a haematology analyser (detection apparatus 2) and contains a result error flag which does not allow for the calculation of the ICIS score.

Similarly, the error code "ICIS_ACTION_MESSAGE" from the haematology analyser (detection apparatus 2) may contain an action message such as "Suspect sample" or "Check the sample" which does not allow for the calculation of the ICIS score.

Above, embodiments have been described in which the blood cell counter system 1 has a detection apparatus 2, first diagnosis support apparatus 3 and a second diagnosis support apparatus 3'. In such a configuration, the first diagnosis support apparatus may evaluate the data of a forward scattered light signal, a side scattered light signal and a fluorescence signal to create corresponding scattergrams and determine the set of haematological parameters for the blood sample of a subject. Subsequently, the second diagnosis support apparatus 3' obtains the plurality of haematological parameters and performs the plausibility check as described above.

According to an alternative embodiment, the blood cell counter system 1 may comprise a detection apparatus 2 and a single diagnosis support apparatus 3. In such a configuration, the diagnosis support apparatus 3 may evaluate the data of a forward scattered light signal, a side scattered light signal and a fluorescence signal to create corresponding scattergrams and determine the set of haematological parameters for the blood sample of a subject to obtain the plurality of haematological parameters and to perform the plausibility check as described above.

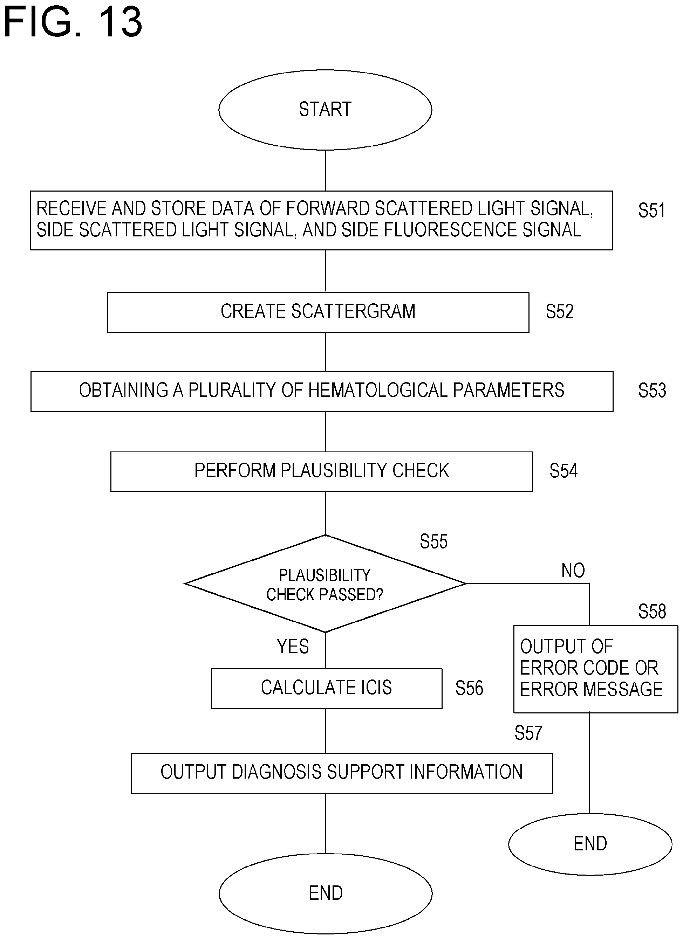

Such an alternative embodiment may be used to implement the method according to the flowchart of Fig. 13. In particular, according to step S51 of Fig. 13, the CPU 31a of the diagnosis support apparatus 3 may receive via the communication interface 31g data of forward scattered light signals, side scattered light signals, and fluorescence signals outputted by the detector 5 of the detection apparatus 2, and store the data in the memory 31b. Subsequently, in step S52, the CPU 31a creates corresponding scattergrams, for example, at least an RET scattergram having a Y-axis of the intensity of forward scattered light signals and an X-axis of the intensity of fluorescence signals, both of which signals are outputted by the detector 5; a DIFF scattergram having a Y-axis of the intensity of fluorescence signals and an X-axis of the intensity of side scattered light signals, both of which signals are outputted by the detector 5; and a WBC/BASO scattergram having a Y-axis of the intensity of forward scattered light signals and an X-axis of the intensity of side scattered light signals, both of which signals are outputted by the detector 5.

Next, the CPU 31a of the diagnosis support apparatus 3 obtains, by using the scattergrams as detection results, a plurality of haematological parameters for the blood of the subject. Examples of such haematological parameters are NEUT#, NE-SFL, RET#, HFLC#, IG#, EO#, Delta-He, RBC-He, PLT (as described above).

Next, the CPU 31a of the diagnosis support apparatus 3 also performs the steps of performing the plausibility check of steps S54 and S55, calculating ICIS index value (step S56), and outputting diagnosis support information (step S57) or outputting an error code or errors message (step S58). Steps S54 - S58 correspond to steps S 32 - S36 of Fig. 6. The skilled person recognizes that all steps of Fig. 13 are performed by the single diagnosis support apparatus 3, in contrast to the embodiment described with regard to Fig. 6 in which the work load of determining the set of haematological parameters and the plausibility check are shared between two diagnosis support apparatuses.

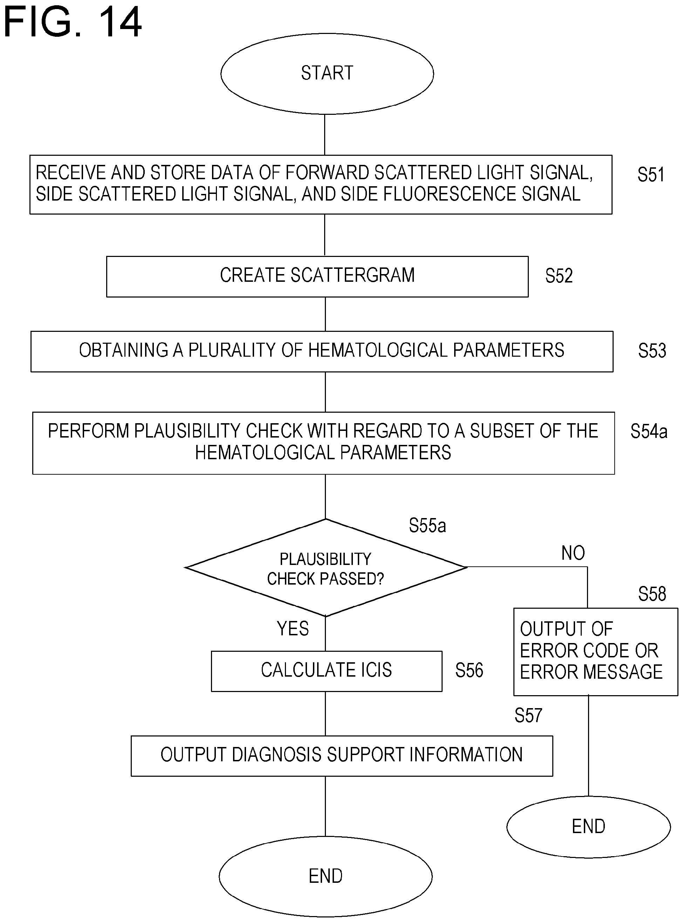

The alternative embodiment may be also be used to implement the method according to the flowchart of Fig. 14. In comparison to Fig. 13, here the plausibility checks of steps S54a and S55a is performed with regard to a subset of the haematological parameters, i.e. not with regard to the complete set of haematological parameters as obtained from the scattergrams in step S53. As explained above, the complete set of haematological parameters as obtained by the single diagnosis support apparatus in step S53 may be as follows: NEUT#, NE-SFL, RET#, HFLC#, IG#, EO#, Delta-He, RBC-He, PLT. In this example, the subset of haematological parameters which is used for the plausibility check may correspond to the three parameters NEUT#, NE-SFL, RET#. Also in the alternative embodiment which applies a single diagnosis support apparatus, using only a subset of the haematological parameters for the plausibility check allows for more efficient reliability check performance.

Above, embodiments have been described in which the blood cell counter system 1 has a detecting apparatus 2 which includes a detector 5 configured to detect multiple types of characteristic information of the blood sample such as forward and side scattered lights and fluorescence from cells after being passed through the flow cell. The detecting apparatus 2 may, additionally or alternatively, include a different detector configured to measure direct current (DC) impedance of cells of a blood sample passing individually through a cell interrogation zone. The detecting apparatus 2 may also or alternatively include a further different detector configured to measure radiofrequency (RF) conductivity of cells of the blood sample passing individually through the cell interrogation zone.

Above, embodiments have been described in which the WBC/BASO detection sample for specifying the basophils (BASO) is prepared by subjecting the blood to a dilution process, and further to a hemolyzation process by use of a dedicated reagent for hemolyzing red blood cells. Alternatively, the detection sample for specifying the basophils (BASO) may be prepared by subjecting the blood to a dilution process, a haemolyzing process and further to a staining process by use of a dedicated reagent for staining blood cells. In that case, a scattergram having a Y-axis of the intensity of forward scattered light signals and an X-axis of the intensity of fluorescence signals may be prepared to perform cell classification.

Above, embodiments have been described in which both of the DIFF scattergram and the WBC/BASO scattergram are analysed for classifying the white blood cells into five groups of monocyte (MONO), lymphocyte (LYMPH), neutrophil (NEUT), basophil (BASO) and eosinophil (EO). Alternatively, only the DIFF scattergram may be analysed for classifying the white blood cells into the five groups.

Above, embodiments have been described in which the ICIS index value is outputted in the output screen when the plausibility check has passed while the ICIS index value is not outputted in the output screen when the plausibility check has failed. Alternatively, the ICIS index value may be outputted in the output screen with a flag indicating unreliability of the ICIS index value when the plausibility check has failed.

Above, embodiments have been described in which the ICIS index value is outputted as the indication of a probability of the infectious response of the subject. Alternatively, a flag or message indicating the probability of the infectious response of the subject may be outputted as the indication.

Above, embodiments have been described in which the haematological parameter NE-SFL is used as a parameter reflecting a quality of a reagent which is mixed with the blood since NE-SFL reflect the degree of staining of cells by the staining reagent. Alternatively or additionally, a haematological parameter reflecting a quality of lysing reagent may be used. For example, a white blood cell count such as monocyte count or neutrophil count may be used as the parameter reflecting a quality of the lysing reagent since an abnormally large number of monocytes or neutrophils will occur in the presence of excessive debris and this is indicative of incomplete lysing.

## Claims

1. A computer-implemented method for testing blood of a subject, comprising:
- obtaining a plurality of haematological parameters for the blood;
- performing a plausibility check of at least one of the haematological parameters; and
- outputting an indication of a determined probability of an infectious response of the subject based on at least one of the parameters according to a result of the plausibility check.

2. The computer-implemented method of claim 1, wherein
the step of performing the plausibility check comprises checking a subset of the haematological parameters.

3. The computer-implemented method of one of claims 1 - 2, wherein
the step of performing the plausibility check comprises checking a haematological parameter obtained from a white blood cell differential measurement.

4. The computer-implemented method of one of claims 1 - 3, wherein
the step of performing the plausibility check comprises checking a white blood cell count as a haematological parameter.

5. The computer-implemented method of one of claims 1 - 4, wherein
the step of performing the plausibility check comprises checking a haematological parameter whose value is related to obtaining a reliable value of another haematological parameter among the plurality of haematological parameters.

6. The computer-implemented method of any one of claims 1 - 5, wherein
the step of performing the plausibility check comprises comparing a value of each of the haematological parameters used for the plausibility check with a corresponding threshold value or a corresponding lower and upper threshold.

7. The computer-implemented method of any one of claims 1 - 6, wherein
the step of outputting the indication according to the result of the plausibility check comprises outputting the indication when the plausibility check has passed and not outputting the indication when the plausibility check has not passed.

8. The computer-implemented method of any one of claims 1 - 7, wherein
when the plausibility check has not passed, a measurement result of at least one haematological parameter which causes a failure of the plausibility check is outputted.

9. The computer-implemented method of one of claims 1 - 8, further comprising
outputting an error message or error code when the plausibility check has not passed.

10. The computer-implemented method of claim 9, wherein
the error message or error code comprises information indicating at least one haematological parameter which causes a failure of the plausibility check.

11. The computer-implemented method of any one of claims 1 - 10, wherein
the indication of the probability of the infectious response comprises a value of an index value corresponding to the probability of the infectious response.

12. The computer-implemented method of any one of claims 1 - 11, further comprising
generating the indication based on at least two of the haematological parameters, wherein
the step of performing the plausibility check comprises checking a part of the at least two of the haematological parameters.

13. The computer-implemented method of any one of claims 1 - 11, further comprising
generating the indication based on at least two of the haematological parameters, wherein
the step of performing the plausibility check comprises checking a part of the at least two haematological parameters and a haematological parameter different from the at least two of the haematological parameters.

14. The computer-implemented method of any one of claims 1 - 13, further comprising
performing a general plausibility check of a detection apparatus that measures the blood.

15. The computer-implemented method of one of claims 1 - 14, wherein
the step of performing the plausibility check comprises checking a haematological parameter reflecting an operational status of a detection apparatus that measures the blood or a haematological parameter reflecting a quality of a reagent which is mixed with the blood.

16. A diagnosis support apparatus comprising:
a controller configured for
- obtaining a plurality of haematological parameters for the blood;
- performing a plausibility check of at least one of the haematological parameters; and
- outputting an indication of a determined probability of an infectious response of the subject based on at least one of the parameters according to a result of the plausibility check.

17. A blood cell counter system comprising a blood detector apparatus and a diagnosis support apparatus according to claim 16.

18. A computer program product, comprising:
a computer readable medium storing instructions to enable a general purpose computer to perform operations of the computer-implemented method of any of claims 1 - 15.
